# FASCICULE DE BREVET EUROPEEN

(11) **EP 2 529 709 B1**
(45) Date de publication et mention de la délivrance du brevet: **16.09.2015**
(21) Numéro de dépôt: 12354032.0
(22) Date de dépôt: 22.05.2012
(51) Int. Cl.: A61F 5/02

(54) **Corset pour lombalgie**
Lombalgiegürtel
Corset for lumbago

(30) Priorité: 31.05.2011 FR 1101676
(43) Date de publication de la demande: 05.12.2012
(73) Titulaire: Chabloz Composants, 38170 Seyssinet-Pariset (FR)
(72) Inventeur: Graf, Henry, 69160 Tassin-la-Demi-Lune (FR); Mariani, Patrice, 69290 Craponne (FR); Chabloz, Pierre, 38450 Saint-Georges-de-Commiers (FR)
(74) Mandataire: Hecké, Gérard

(56) Documents cités:
- FR-A1- 2 085 624
- FR-A1- 2 872 407
- US-A- 5 012 798
- US-A1- 2004 147 861
- US-A1- 2007 156 073

## Description

### Domaine technique de l'invention

L'invention concerne un corset pour lombalgie comportant :
- une bande lombaire destinée à prendre appui contre la chaîne lombaire d'un patient, tout en laissant libre la chaîne dorsale, ainsi que le sacrum et le fessier de ce patient, cette bande lombaire étant pourvue de moyens de mise en lordose du patient,
- deux coques latérales,
- des moyens déformables de fixation, s'étendant à partir des coques latérales et destinés à prendre appui contre la région abdominale du patient,
- deux mâts s'étendant à partir des coques latérales, les extrémités libres de ces mâts étant destinées à prendre appui contre une région pectorale du patient, chaque mât comprenant une portion intermédiaire et une portion terminale et,
- des moyens de rappel élastique, propres à relier les extrémités libres desdits mâts.

### État de la technique

La plupart des corsets de l'état de la technique sont fondés sur un traitement de la lombalgie tendant à réduire la cambrure du patient. Les corsets connus sont classiquement conformés pour induire une bascule du bassin du patient et ainsi diminuer la pression exercée sur les facettes articulaires, devenues arthrosiques. Néanmoins, ces corsets se révèlent lourds et recouvrent une large partie du corps du patient, induisant une gêne dans les mouvements du patient et à l'égard de la chaleur. Des corsets pour lombalgie sont connus, par exemple, des documents FR-2085624 et US-2007/0156073.

Pour remédier aux inconvénients de l'art antérieur évoqués ci-dessus, le document FR-B-2872407 propose un corset pour lombalgie qui prend le contre-pied de l'art antérieur en tenant compte de l'aspect musculaire et non osseux.

Comme représenté à la figure 1, le document FR-B-2872407 décrit un corset pour lombalgie comprenant :
- une bande lombaire (2), destinée à prendre appui contre la chaîne lombaire d'un patient, tout en laissant libre la chaîne dorsale, ainsi que le sacrum et le fessier de ce patient, cette bande lombaire (2) étant pourvue de moyens (2₃) de mise en lordose du patient,
- deux coques latérales (4, 6), dont chacune est destinée à être rendue solidaire du trochanter du patient;
- des moyens (10, 12, 14) déformables de fixation, s'étendant à partir des coques latérales (4, 6) et destinés à prendre appui contre la région abdominale du patient,
- deux mâts (16, 18) sensiblement rigides, s'étendant à partir des coques latérales (4, 6), les extrémités libres de ces mâts (16, 18) étant destinées à prendre appui contre une région pectorale du patient et,
- des moyens (20) de rappel élastique, propres à relier les extrémités libres (16_{3,} 18₃) desdits mâts (16, 18).

En libérant le bassin du patient et en favorisant la mise en lordose, le corset décrit ci-dessus résout les principaux inconvénients de l'art antérieur. Cette solution connue reste, néanmoins, coûteuse car le corset doit être adapté à la morphologie du patient, pour produire un effet curatif optimisé.

### Objet de l'invention

L'invention a pour but un corset pour lombalgie remédiant aux inconvénients de l'art antérieur.

En particulier, l'invention a pour but un corset, peu encombrant et confortable, adaptable à une large gamme de morphologie de patients et traitant efficacement la lombalgie, en soulageant significativement le patient moyennant le port dudit corset.

L'invention a, notamment, pour but d'améliorer le corset décrit dans le document FR-B-2872407.

Selon l'invention, ce but est atteint par un corset pour lombalgie tel que défini dans la revendication indépendante 1. Différents développements de l'invention sont définis dans les revendications dépendantes.

Selon un développement de l'invention, chaque coque latérale est montée, indépendamment, mobile selon un axe latéral xx' et selon un axe pivot, (P) ou (P'), sur les moyens de solidarisation sélective et les moyens de solidarisation sélective sont pourvus d'un organe de verrouillage des coques latérales en position.

Selon un mode de réalisation préférentiel, chaque mât a une portion de moindre résistance située entre la portion intermédiaire et la portion terminale de manière à permettre l'appui, au moins partiellement, de la portion terminale correspondante dudit mât, contre la région sous axillaire-pectorale du patient, en position portée du corset.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :

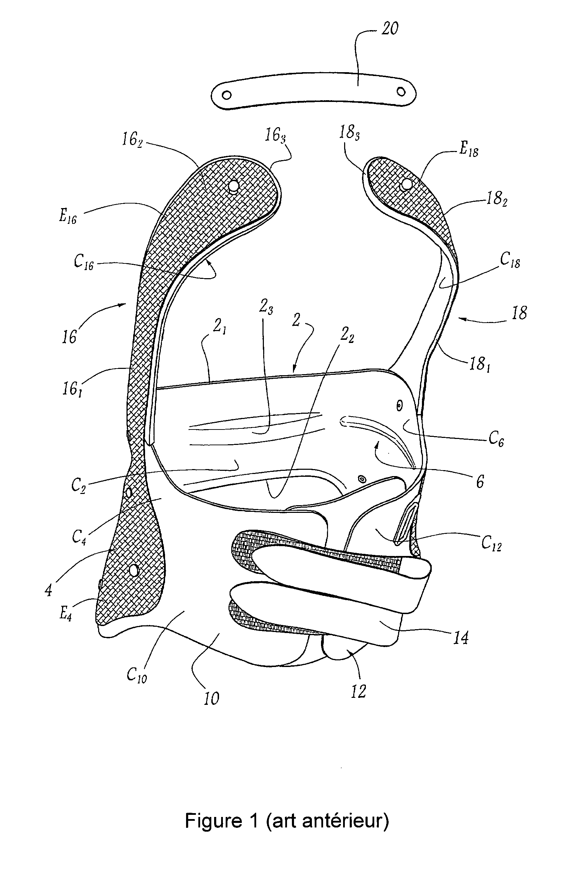

- La figure 1 représente, schématiquement et en perspective, un corset pour lombalgie selon l'art antérieur,
- La figure 2 représente schématiquement et, en perspective et en vue de face, un corset pour lombalgie selon un mode de réalisation particulier de l'invention,
- La figure 3 représente, schématiquement et en vue de dos, le corset pour lombalgie selon la figure 2,
- La figure 4 représente, schématiquement, un agrandissement du corset selon la figure 2.
- La figure 5 représente, schématiquement, une vue isolée de face d'un mât d'un corset pour lombalgie selon un mode de réalisation particulier de l'invention.
- La figure 6 représente, schématiquement, une vue isolée de profil du mât selon la figure 5.
- La figure 7 représente, schématiquement et en vue de profil, un corset selon la figure 2.

### Description de modes particuliers de réalisation

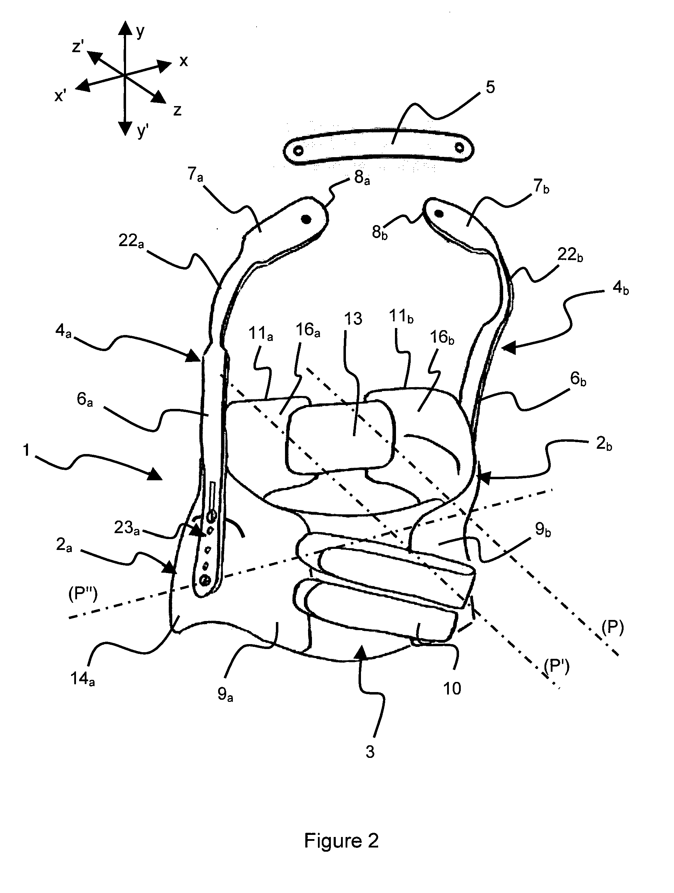

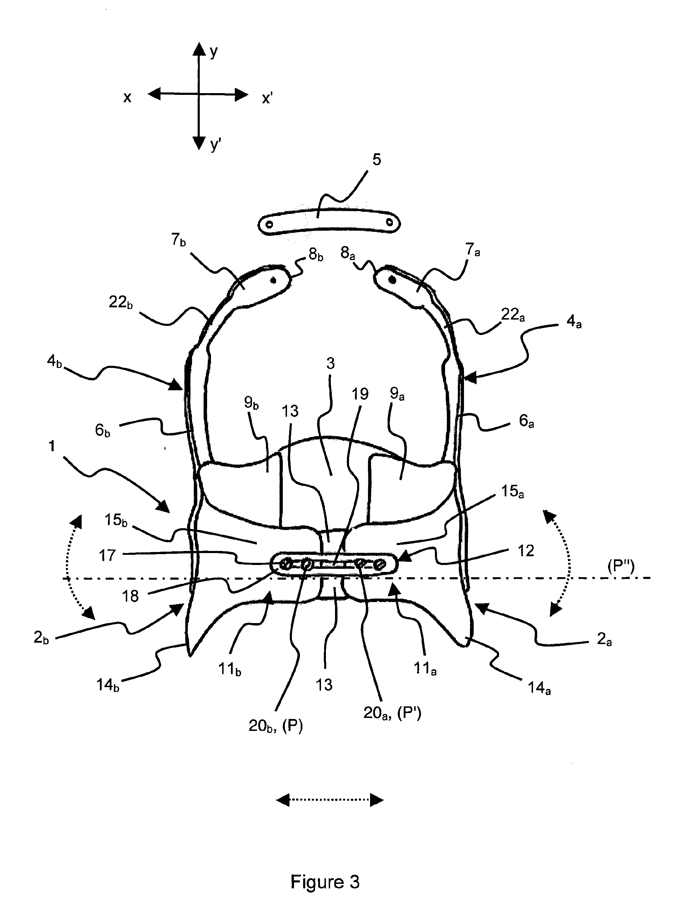

Selon un mode particulier de réalisation représenté aux figures 2 et 3, un corset pour lombalgie comporte une bande lombaire 1, deux coques latérales, respectivement 2ₐ et 2_{b}, des moyens 3 déformables de fixation, deux mâts, respectivement 4ₐ et 4_{b}, et des moyens 5 de rappel élastique.

Les moyens 3 déformables de fixation s'étendent à partir des coques latérales, 2ₐ et 2_{b}, et sont destinés à prendre appui contre la région abdominale du patient.

Les deux mâts, 4ₐ et 4_{b}, s'étendent vers le haut, à partir des coques latérales, 2ₐ et 2_{b}, respectives. Chaque mât, 4ₐ ou 4_{b}, comprend une portion intermédiaire, 6ₐ ou 6_{b}, et une portion terminale, 7ₐ ou 7_{b}. Les portions intermédiaires, 6ₐ et 6_{b}, sont destinées à prendre appui contre les flancs du patient et les portions terminales, 7ₐ ou 7_{b}, contre les muscles pectoraux du patient.

Les extrémités libres, 8ₐ et 8_{b}, des mâts, 4ₐ et 4_{b}, sont destinées à prendre appui contre la région pectorale du patient, en particulier sous-claviculaire, et sont reliées mutuellement par les moyens 5 de rappel élastique. Les moyens 5 de rappel élastique peuvent être un lien élastique, par exemple, une bande de tissu élastique.

La bande lombaire 1 est conformée pour être en contact étroit avec la chaîne lombaire du patient tout en dégageant le fessier et la chaîne dorsale. Ainsi, la bande lombaire 1 est destinée à prendre appui contre la chaîne lombaire du patient, tout en laissant libre la chaîne dorsale, ainsi que le sacrum et le fessier de ce patient. Lorsque le corset est porté par le patient, la bande lombaire 1 prend, de préférence, appui sur la chaîne lombaire du patient en se trouvant centrée au voisinage de la deuxième ou troisième vertèbre lombaire.

Chaque coque latérale, 2ₐ ou 2_{b}, est reliée à l'opposé de la bande lombaire 1 par les moyens 3 déformables de fixation. Comme représenté à la figure 2, chaque coque latérale, 2ₐ ou 2_{b}, est prolongée par un retour antérieur, 9ₐ ou 9_{b}, respectif. Les deux retours antérieurs, 9ₐ et 9_{b}, sont, avantageusement, solidarisés par l'intermédiaire de sangles réglables 10 selon tout procédé connu. Les moyens 3 déformables de fixation sont destinés à prendre appui contre la région abdominale du patient.

Comme représenté aux figures 2 et 3, la bande lombaire 1 est formée par deux zones de maintien lombaire, 11ₐ et 11_{b}, des moyens 12 de solidarisation sélective des deux coques latérales, 2ₐ et 2_{b}, dans différentes positions et des moyens 13 de mise en lordose du patient.

Chacune des coques latérales, 2ₐ et 2_{b}, comporte respectivement une zone de maintien lombaire, 11ₐ et 11_{b}, et une zone latérale, 14ₐ et 14_{b,} destinée à être rendue solidaire du trochanter du patient. Chaque coque latérale, 2ₐ ou 2_{b}, présente une face externe, 15ₐ ou 15_{b,} respective et une face interne, 16ₐ ou 16_{b,} respective. En position portée du corset sur le patient, les zones de maintien lombaire, 11ₐ et 11_{b,} sont destinées à prendre appui sur la région lombaire du patient par les faces internes, 16ₐ et 16_{b} (figure 2).

Ainsi, les zones de maintien lombaire, 11ₐ et 11_{b}, les moyens 12 de solidarisation sélective, les coques latérales, 2ₐ et 2_{b}, ainsi que les moyens 13 de mise en lordose constituent un ensemble solidaire apte à être en contact ferme avec le patient pour favoriser la mise en lordose naturelle du patient.

Chaque coque latérale, 2ₐ et 2_{b}, est avantageusement montée mobile, indépendamment, sur les moyens 12 de solidarisation sélective selon un axe latéral xx' et selon un axe pivot, (P') ou (P), respectif. Chaque coque latérale, 2ₐ et 2_{b}, peut être disposée dans différentes positions, de façon indépendante et de manière à stabiliser parfaitement le bassin dans un plan frontal et/ou sagittal. On entend par plan sagittal, un plan médian, vertical et dans le sens antéropostérieur c'est-à-dire d'avant en arrière. On entend par plan frontal, un plan vertical et perpendiculaire au plan sagittal.

Comme représenté à la figure 3, les moyens 12 de solidarisation sélective sont, avantageusement, pourvus d'un organe de verrouillage 17 des coques latérales, 2ₐ et 2_{b}, en position. Les moyens 12 de solidarisation sélective peuvent être constitués par une coulisse 18 munie de l'organe de verrouillage 17 et de deux pièces mobiles, 20ₐ et 20_{b}. Chacune des pièces, 20ₐ et 20_{b}, est fixée à demeure à la zone de maintien lombaire, 11ₐ et 11_{b,} respective.

La coulisse 18 peut se présenter sous la forme d'une barrette pourvue dans sa partie médiane d'une lumière 19.

La coulisse 18 comprend les deux pièces mobiles, 20ₐ et 20_{b}, par exemple des vis à tête de type rivet, engagées dans la lumière 19 de manière à mettre chacune des têtes en butée sur la coulisse 18. L'extrémité opposée de chacune des têtes est fixée, à demeure, à la zone de maintien lombaire, 11ₐ ou 11_{b}, qui lui est associée (figure 3).

Les moyens de verrouillage 17 peuvent être constitués par au moins deux vis de blocage engagés dans la lumière 19 pour maintenir la barrette solidaire des coques latérales, 2ₐ et 2_{b}, par vissage.

Chaque pièce mobile, 20ₐ et 20_{b}, forme respectivement l'axe pivot, (P') ou (P), de la coque latérale, 2ₐ ou 2_{b}, correspondante à laquelle elle est fixée.

Ainsi, comme illustré à la figure 3, les coques latérales, 2ₐ ou 2_{b}, peuvent basculer indépendamment l'une de l'autre, autour de leur axe pivot, (P') ou (P), jusqu'à une position finale sélectionnée (voir les flèches courbes pointillées à droite et à gauche de la figure 3). Cette position finale correspond à un appui maximal de la bande lombaire 1 sur le patient.

La lumière 19 de la barrette est également conformée pour permettre un déplacement latéral selon l'axe xx' (voir flèches droites pointillées à la figure 3) et le rapprochement ou l'éloignement des coques latérales, 2ₐ et 2_{b}, entre-elles.

Une fois les coques latérales, 2ₐ et 2_{b}, positionnées, l'organe de verrouillage 17 est actionné de manière à bloquer chacune des coques latérales, 2ₐ et 2_{b}, dans la position finale sélectionnée.

Les moyens 12 de solidarisation sélective permettent ainsi l'adaptation du corset à la morphologie du patient et l'ajustement de chacune des coques latérales, 2ₐ et 2_{b}, transversalement selon l'axe xx' et en rotation autour de leur axe pivot, (P') et (P), respectif. Ainsi, selon la largeur du bassin du patient et le marquage plus ou moins prononcé de la taille du patient, chaque coque latérale, 2ₐ ou 2_{b}, peut être basculée indépendamment vers le haut ou le bas. L'ajustement du corset est alors optimisé pour un effet amélioré du traitement.

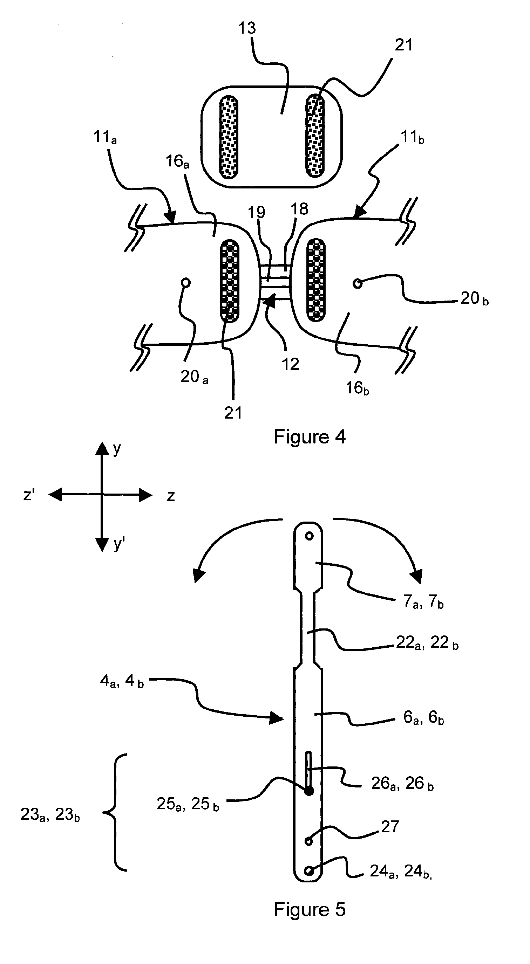

Comme représenté à la figure 4, les moyens 13 de mises en lordose du patient sont fixés aux faces internes, 16ₐ et 16_{b}, respectives des zones de maintien lombaire, 11ₐ ou 11_{b}, par des moyens 21 d'attache amovible. Les moyens 21 d'attache amovible sont, avantageusement, des bandes auto-agrippantes de type Velcro® permettant une accroche amovible et temporaire.

À titre d'exemple, les moyens 13 de mise en lordose du patient peuvent être constitués par au moins un coussin paravertébral. La face interne, 16ₐ ou 16_{b,} de chaque zone de maintien, 11ₐ ou 11_{b,} comporte une première bande auto-agrippante. Le coussin paravertébral est muni d'au moins une seconde bande auto-agrippante complémentaire, de préférence deux bandes auto-agrippantes. La seconde bande auto-agrippante est propre à coopérer avec au moins une des premières bandes auto-agrippantes des faces internes, 16ₐ ou 16_{b,} pour former les moyens 21 d'attache amovible.

Selon un mode de réalisation préférentiel représenté aux figures 3 à 7, chaque mât, 4ₐ ou 4_{b}, a une portion de moindre résistance, 22ₐ ou 22_{b}, située entre la portion intermédiaire, 6ₐ ou 6_{b}, et la portion terminale, 7ₐ ou 7_{b}, correspondante de manière à permettre l'appui, au moins partiellement, de la portion terminale, 7ₐ ou 7_{b}, du mât respectif, 4ₐ ou 4_{b}, contre la région sous axillaire-pectorale du patient, en position portée du corset.

Les mâts latéraux, 4ₐ et 4_{b}, sont, avantageusement, constitués par un matériau malléable. Les mâts latéraux, 4ₐ et 4_{b}, sont, de préférence, en aluminium.

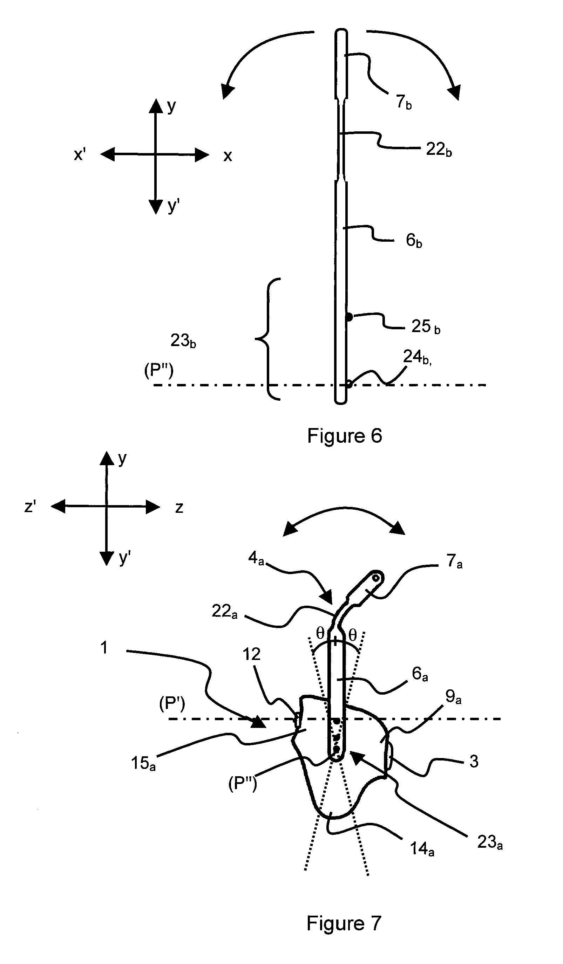

Comme représenté aux figures 5 et 6, le mât latéral, 4ₐ ou 4_{b}, est, de préférence, un élément plat rectiligne, de section principalement rectangulaire présentant la portion de moindre résistance, 22ₐ ou 22_{b}. La portion de moindre résistance, 22ₐ ou 22_{b}, est constituée par une portion amincie du mât, 4ₐ ou 4_{b}. L'amincissement de la portion de moindre résistance, 22ₐ ou 22_{b}, peut être réalisé selon l'épaisseur et/ou la largeur du mât, 4ₐ ou 4_{b}, correspondant.

Ainsi, comme illustré par les flèches courbes pleines aux figures 5 et 7, l'amincissement dans la largeur permet de galber le mât, 4ₐ ou 4_{b}, selon l'axe sagittal.

Comme illustré par les flèches pleines aux figures 6 et 7, l'amincissement dans l'épaisseur permet de galber le mât, 4ₐ ou 4_{b}, latéral de manière à plaquer la portion terminale, 7ₐ ou 7_{b}, correspondante, contre la région sous axillaire-pectorale du patient, en position portée du corset.

En outre, chaque mât, 4ₐ ou 4_{b}, peut être fixé à la coque latérale, 2ₐ ou 2_{b}, respective aux moyens d'une liaison réglage, 23ₐ ou 23_{b}, respective de manière mobile en translation selon un axe vertical yy' c'est-à-dire de haut en bas et/ou en rotation selon un second axe pivot (P"). La liaison réglage, 23ₐ ou 23_{b}, est propre à ajuster la position d'appui sous-claviculaire de la portion terminale, 7ₐ ou 7_{b}, correspondante du mat, 4ₐ ou 4_{b}, en fonction de la morphologie du patient. Le second axe pivot (P") permet de couvrir, avantageusement, un angle θ par rapport à un axe yy' compris entre -15° et +15° (figure 7).

La translation selon un axe vertical yy' permet d'orienter indépendamment les mâts, 4ₐ et 4_{b}, dans l'axe du tronc c'est-à-dire de haut en bas afin d'ajuster le corset selon la taille en hauteur du patient.

La rotation selon le second axe pivot (P") permet d'orienter les mâts, 4ₐ ou 4_{b}, indépendamment, dans le plan sagittal.

Comme illustré aux figures 2, 3 et 7, la liaison réglage, 23ₐ ou 23_{b}, peut être réalisée selon un principe similaire à celui des moyens 12 de solidarisation sélective, c'est-à-dire par réalisation du second axe pivot (P"), par exemple, grâce à une première fixation, 24ₐ ou 24_{b}, de type rivet et un ensemble rivet/insert, 25ₐ ou 25_{b}, logé dans un organe de guidage, 26ₐ ou 26_{b} respectif. L'organe de guidage, 26ₐ ou 26_{b}, est conformé de façon à permettre un mouvement en translation, pour le réglage selon l'axe yy', et en rotation, pour le basculement du mât, 4ₐ ou 4_{b}, dans le plan sagittal. Le verrouillage de la position finale sélectionnée peut alors être réalisé selon tout procédé connu, par exemple, par vissage de l'ensemble rivet/insert, 25ₐ ou 25_{b}. Le verrouillage est alors effectué par simple visage de l'ensemble rivet/insert, 25ₐ ou 25_{b}. Plusieurs orifices 27 destinés à accueillir un rivet pour former le second axe pivot (P"), peuvent être réalisés sous la portion intermédiaire, 6ₐ ou 6_{b}, de chaque mât, 4ₐ ou 4_{b}. Ces orifices 27 permettent d'ajuster la position du mât, 4ₐ ou 4_{b}, en fonction de l'axe yy'.

L'invention n'est pas limitée aux exemples décrits ci-dessus. En particulier, les moyens 12 de solidarisation sélective et les liaisons réglages, 23ₐ et 23_{b}, peuvent être réalisés selon tout procédé connu, par exemple, en utilisant des éléments de verrouillage par encliquetage.

Par ailleurs, le corset selon l'invention peut être revêtu par un gainage approprié améliorant le confort du corset et sa mise en place. Le gainage peut, classiquement, être constitué par un revêtement en néoprène ou par un enrobage de renfort tel que décrit dans le brevet FR-B-2872407.

Le corset selon l'invention est remarquable en ce qu'il est réglable et adaptable à la morphologie du patient tout en présentant une efficacité améliorée. Le corset selon l'invention est peu couvrant et, par conséquent, facilement supportable par le patient. En outre, le corset selon l'invention s'ajuste au plus près du corps du patient pour une efficacité optimisée. Un tel corset permet d'éviter une fabrication sur mesure, longue, fastidieuse et coûteuse.

## Revendications

1. Corset pour lombalgie comportant:
- une bande lombaire (1) destinée à prendre appui contre la chaîne lombaire d'un patient, tout en laissant libre la chaîne dorsale, ainsi que le sacrum et le fessier de ce patient,
- deux coques latérales (2ₐ, 2_{b}) dont chacune comporte une zone de maintien lombaire (11ₐ, 11_{b}) présentant une face externe (15ₐ, 15_{b}) et une face interne (16ₐ, 16_{b}), lesdites zones (11ₐ, 11_{b}) étant destinées à prendre appui sur la région lombaire par lesdites faces internes (16a, 16b), en position portée du corset,
- des moyens (13) de mise en lordose du patient fixés aux faces internes (16ₐ, 16_{b}) desdites zones (11ₐ, 11_{b}) par des moyens (21) d'attache amovible,
- des moyens (12) de solidarisation sélective des deux coques latérales (2a, 2b) via lesquels lesdites coques latérales (2ₐ, 2_{b})sont aptes à être sélectivement solidarisées dans différentes positions, ladite bande lombaire (1) étant formée par les deux zones (11ₐ, 11_{b}) de maintien lombaire, les moyens (12) de solidarisation sélective des coques latérales (2a, 2b) et les moyens (13) de mise en lordose du patient, le corset comportant en outre :
- des moyens (3) déformables de fixation, s'étendant à partir des coques latérales (2ₐ, 2_{b}) et destinés à prendre appui contre la région abdominale du patient,
- deux mâts (4ₐ, 4_{b}) s'étendant à partir des coques latérales (2ₐ, 2_{b}), les extrémités libres (8ₐ, 8_{b}) de ces mâts (4ₐ, 4_{b}) étant destinées à prendre appui contre une région pectorale du patient, chaque mât (4ₐ, 4_{b}), comprenant une portion intermédiaire (6ₐ, 6_{b}) et une portion terminale (7ₐ, 7_{b}) et,
- des moyens (5) de rappel élastique, propres à relier les extrémités libres (8ₐ, 8_{b}) desdits mâts (4ₐ, 4_{b}).

2. Corset selon la revendication 1, dans lequel chaque coque latérale (2ₐ, 2_{b}) est montée, indépendamment, mobile selon un axe latéral xx' et selon un axe pivot, P ou P', sur les moyens (12) de solidarisation sélective et dans lequel les moyens (12) de solidarisation sélective sont pourvus d'un organe de verrouillage (17) des coques latérales (2ₐ, 2_{b}) en position.

3. Corset selon la revendication 2, dans lequel les moyens (12) de solidarisation sélective sont constitués par une coulisse (18) munie de l'organe de verrouillage (17) et de deux pièces mobiles (20ₐ, 20_{b}), chacune desdites pièces (20ₐ, 20_{b}) étant fixée à demeure à la zone de maintien lombaire (11ₐ, 11_{b}) respective.

4. Corset selon la revendication 3, dans lequel chaque pièce mobile (20ₐ, 20_{b}) forme l'axe pivot, P ou P', de la coque latérale (2ₐ, 2_{b}) correspondante à laquelle elle est fixée.

5. Corset selon l'une quelconque des revendications 1 à 4, dans lequel chaque mât (4ₐ, 4_{b}), a une portion de moindre résistance (22ₐ, 22_{b}), située entre la portion intermédiaire (6ₐ, 6_{b}) et la portion terminale (7ₐ, 7_{b}) de manière à permettre l'appui, au moins partiellement, de la portion terminale (7ₐ, 7_{b}) correspondante dudit mât (4ₐ, 4_{b}), contre la région sous axillaire-pectorale du patient, en position portée du corset.

6. Corset selon la revendication 5, dans lequel la portion de moindre résistance (22ₐ, 22_{b}) est constituée par une portion amincie du mât (4ₐ, 4_{b}).

7. Corset selon l'une quelconque des revendications 1 à 6, dans lequel chaque mât (4ₐ, 4_{b}), est fixé aux moyens d'une liaison réglage (23ₐ, 23_{b}) à la coque latérale (2ₐ, 2_{b}) respective, de manière mobile en translation selon un axe vertical yy' et/ou en rotation selon un second axe pivot P" couvrant un angle θ par rapport à l'axe yy' compris entre -15° et +15°, ladite liaison réglable (23ₐ, 23_{b}) étant propre à ajuster la position d'appui sous claviculaire de la portion terminale (7ₐ, 7_{b}) correspondante dudit mat (4ₐ, 4_{b}), en fonction de la morphologie du patient.

8. Corset selon l'une quelconque des revendications 1 et 7, dans lequel les mâts (4ₐ, 4_{b}), sont en aluminium.

9. Corset selon l'une quelconque des revendications 1 à 8, dans lequel les moyens (13) de mise en lordose du patient sont constitués par au moins un coussin paravertébral.

10. Corset selon la revendication 9, dans lequel la face interne (16ₐ, 16_{b}) de chaque zone de maintien lombaire (11ₐ, 11_{b}) comporte une première bande auto-agrippante et dans lequel le coussin paravertébral est muni d'au moins une seconde bande auto-agrippante propre à coopérer avec au moins une des premières bandes auto-agrippantes desdites faces internes (16ₐ, 16_{b}), pour former les moyens (21) d'attache amovible.

## Patentansprüche

1. Korsett für Lumbalgien, das umfasst:
- ein Lumbalband (1), das dazu bestimmt ist, an die Lumbalkette eines Patienten anzuliegen, wobei die Rumpfkette freigelassen wird, ebenso wie das Kreuzbein und der Gesäf3muskel dieses Patienten,
- zwei seitliche Schalen (2a, 2b), die beide einen Lumbalstützbereich (11 a, 11b) umfassen und eine Außenseite (15a, 15b) und eine Innenseite (16a, 16b) umfassen, wobei die genannten Bereiche (11a, 11b) dazu bestimmt sind, im getragenen Zustand des Korsetts mit den genannten Innenseiten (16a, 16b) am Lumbalbereich anzuliegen,
- Mittel (13), mit denen der Patient ins Hohlkreuz gebracht werden soll, die mittels abnehmbarer Befestigungsmittel (21) an den Innenseiten (16a, 16b) der genannten Bereiche (11 a, 11 b) befestigt sind,
- Mittel (12) zur selektiven Befestigung der beiden seitlichen Schalen (2a, 2b), über die die beiden seitlichen Schalen (2a, 2b) in unterschiedlichen Positionen selektiv fest verbunden werden können, wobei das Lumbalband (1) von den beiden Lumbal-Stütz-Bereichen (11a, 11b), den Mitteln (12) zur selektiven Befestigung der Seitenschalen (2a, 2b) und den Mitteln (13) zum Versetzen des Patienten ins Hohlkreuz gebildet wird, wobei das Korsett ferner umfasst:
- verformbare Befestigungsmittel (3), die von den seitlichen Schalen (2a, 2b) ausgehen und dazu bestimmt sind, an die Bauchregion des Patienten anzuliegen,
- zwei Stäbe (4a, 4b), die von den seitlichen Schalen (2a, 2b) ausgehen, wobei die freien Enden (8a, 8b) dieser Stäbe (4a, 4b) dazu bestimmt sind, an den Brustbereich des Patienten anzuliegen, wobei jeder Stab (4a, 4b) einen Zwischenbereich (6a, 6b) und einen Endbereich (7a, 7b) umfasst, und
- Mittel (5) zum elastischen Zurückholen, die geeignet sind, die beiden freien Enden (8a, 8b) der Stäbe (4a, 4b) miteinander zu verbinden.

2. Korsett nach Anspruch 1, bei dem jede seitliche Schale (2a, 2b) gemäß einer seitlichen Achse xx' und gemäß einer Drehachse, P oder P', an den Mitteln zur selektiven Befestigung (12) unabhängig beweglich montiert ist, und bei dem die Mittel zur selektiven Befestigung (12) mit einem Verriegelungselement (17) für die seitlichen Schalen (2a, 2b) in ihrer Position versehen sind.

3. Korsett nach Anspruch 2, bei dem die Mittel zur selektiven Befestigung (12) von einer Gleitschiene (18) gebildet werden, die mit dem Verriegelungselement (17) und zwei mobilen Teilen (20a, 20b) versehen ist, wobei jedes dieser Teile (20a, 20b) dauerhaft an dem jeweiligen Lumbalstützbereich (11a, 11b) befestigt ist.

4. Korsett nach Anspruch 3, bei dem jedes bewegliche Teil (20a, 20b) die Drehachse P bzw. P' der entsprechenden seitlichen Schale (2a, 2b) bildet, an der es befestigt ist.

5. Korsett nach einem der Ansprüche 1 bis 4, bei dem jeder Stab (4a, 4b) einen Bereich geringerer Widerstandsfähigkeit (22a, 22b) aufweist, der sich zwischen dem Zwischenbereich (6a, 6b) und dem Endbereich (7a, 7b) befindet, sodass er, wenn der Patient das Korsett trägt, zumindest teilweise das Anliegen des entsprechenden Endbereichs (7a, 7b) des Stabs (4a, 4b) im Bereich unter den Achseln und in Höhe der Brust des Patienten ermöglicht.

6. Korsett nach Anspruch 5, bei dem der Bereich mit geringerer Widerstandsfähigkeit (22a, 22b) von einem dünneren Bereich des Stabs (4a, 4b) gebildet wird.

7. Korsett nach einem der Ansprüche 1 bis 6, bei dem jeder Stab (4a, 4b) mittels einer regulierbaren Verbindung (23a, 23b) an der jeweiligen seitlichen Schale (2a, 2b) translationsbeweglich gemäß einer vertikalen Achse yy' und/oder drehbeweglich gemäß einer zweiten Drehachse P" befestigt ist, die einen Winkel 9 bezüglich der Achse yy' abdeckt, der -15 bis +15° beträgt, welche regulierbare Verbindung (23a, 23b) geeignet ist, die Position des Anliegens des entsprechenden Endbereichs (7a, 7b) des Stabs (4a, 4b) unter dem Schlüsselbein angepasst an die Körperform des Patienten einzustellen.

8. Korsett nach einem der Ansprüche 1 bis 7, bei dem die Stäbe (4a, 4b) aus Aluminium bestehen.

9. Korsett nach einem der Ansprüche 1 bis 8, bei dem die Mittel (13) zum Versetzen des Patienten ins Hohlkreuz von mindestens einem paravertebralen Kissen gebildet werden.

10. Korsett nach Anspruch 9, bei dem die Innenseite (16a, 16b) jedes Lumbalstützbereichs (11a, 11b) ein erstes Klettband umfasst und das paravertebrale Kissen mit mindestens einem zweiten Klettband versehen ist, das geeignet ist, mit mindestens einem der ersten Klettbänder der Innenseiten (16a, 16b) zusammenzuwirken, um die abnehmbaren Befestigungsmittel (21) zu bilden.

## Claims

1. A corset for lower back pain comprising:
- a lumbar strip (1) designed to press against the lumbar trunk of a patient, while leaving the dorsal trunk and the sacrum and buttocks of this patient free,
- two lateral shells (2ₐ, 2_{b}) each of which comprises a lumbar support zone (11ₐ, 11_{b}) presenting an outer surface (15ₐ, 15_{b}) and an inner surface (16ₐ, 16_{b}), said zones (11 ₐ, 11_{b}) being designed to press on the lumbar region via said inner surfaces (16ₐ, 16_{b}) in the worn position of the corset,
- means (13) for placing the patient in lordosis fixed to the inner surfaces (16ₐ, 16_{b}) of said zones (11ₐ, 11_{b}) by removable attachment means (21),
- means (12) for selective securing of the two lateral shells (2ₐ, 2_{b}) via which said lateral shells (2ₐ, 2_{b}) are able to be selectively secured in different positions,
said lumbar strip (1) being formed by the two lumbar support zones (11ₐ, 11_{b}), the means (12) for selective securing of the lateral shells (2ₐ, 2_{b}) and the means (13) for placing the patient in lordosis,
the corset further comprising,
- deformable fixing means (3) extending from the lateral shells (2ₐ, 2_{b}) and designed to press against the abdominal region of the patient,
- two struts (4ₐ, 4_{b}) extending from the lateral shells (2ₐ, 2_{b}), the free ends (8ₐ, 8_{b}) of these struts (4ₐ, 4_{b}) being designed to press against a pectoral region of the patient, each strut (4ₐ, 4_{b}) comprising an intermediate portion (6ₐ, 6_{b}) and a terminal portion (7ₐ, 7_{b}) and,
- flexible return means (5) designed to connect the free ends (8ₐ, 8_{b}) of said struts (4ₐ, 4_{b}).

2. The corset according to claim 1, wherein each lateral shell (2ₐ, 2_{b}) is fitted, independently, movable along a lateral axis xx' and around an axis formed by a pivot-pin, P or P', on the means (12) for selective securing and wherein the means (12) for selective securing are provided with locking means (17) for securing the lateral shells (2ₐ, 2_{b}) in position.

3. The corset according to claim 2, wherein the means (12) for selective securing are formed by a slide (18) provided with the locking means (17) and with two moving parts (20ₐ, 20_{b}), each of said parts (20ₐ, 20_{b}) being fixed in permanent manner to the respective lumbar support zone (11ₐ, 11_{b}).

4. The corset according to claim 3, wherein each moving part (20ₐ, 20_{b}) forms the pivot-pin, P or P', of the corresponding lateral shell (2ₐ, 2_{b}) whereto it is fixed.

5. The corset according to one of claims 1 to 4, wherein each strut (4ₐ, 4_{b}) has a portion of lesser resistance (22ₐ, 22_{b}) situated between the intermediate portion (6ₐ, 6_{b}) and the terminal portion (7ₐ, 7_{b}) so as to enable the corresponding terminal portion (7ₐ, 7_{b}) of said strut (4ₐ, 4_{b}) to press at least partially against the sub-axillary-pectoral region of the patient in the worn position of the corset.

6. The corset according to claim 5, wherein the portion of lesser resistance (22ₐ, 22_{b}) is formed by a thinned portion of the strut (4ₐ, 4_{b}).

7. The corset according to any one of claims 1 to 6, wherein each strut (4ₐ, 4_{b}) is fixed by means of an adjustable link (23ₐ, 23_{b}) to the respective lateral shell (2ₐ, 2_{b}) in movable manner in translation along a vertical axis yy' and/or in rotation around a second pivot-pin P" covering an angle θ with respect to the axis yy' comprised between -15° and +15°, said adjustable link (23ₐ, 23_{b}) being designed so as to be able to adjust the sub-clavicular pressing position of the corresponding terminal portion (7ₐ, 7_{b}) of said strut (4ₐ, 4_{b}) to suit the morphology of the patient.

8. The corset according to any one of claims 1 to 7, wherein the struts (4ₐ, 4_{b}) are made from aluminium.

9. The corset according to any one of claims 1 to 8, wherein the means (13) for placing the patient in lordosis are formed by at least one paravertebral cushion.

10. The corset according to claim 9, wherein the inner surface (16ₐ, 16_{b}) of each lumbar support zone (11ₐ, 11_{b}) comprises a first self-adhesive strip and wherein the paravertebral cushion is provided with at least a second self-adhesive strip designed to collaborate with at least one of the first self-adhesive strips of said inner surfaces (16ₐ, 16_{b}) to form the removable attachment means (21).
